# EUROPEAN PATENT APPLICATION

(11) **EP 3 150 621 A1**
(43) Date of publication of application: **05.04.2017**
(21) Application number: 15799598.6
(22) Date of filing: 29.05.2015
(51) Int. Cl.: C07K 7/56, C07K 1/02, A61K 38/12, A61P 31/10

(54) **COMPOSITION OF CYCLIC PEPTIDE COMPOUND, PREPARATION METHOD FOR SAME, AND USES THEREOF**

(30) Priority: 29.05.2014 CN 201410235507
(71) Applicant: Shanghai Techwell Biopharmaceutical Co., Ltd, Shanghai 201108 (CN)
(72) Inventor: LIU, Shidong, Shanghai 201108 (CN); WANG, Xiusheng, Shanghai 201108 (CN); JI, Xiaoming, Shanghai 201108 (CN)
(74) Representative: Lavoix
(86) International application number: PCT/CN2015/080229
(87) International publication number: WO 2015/180681

(57) **Abstract**

Disclosed is a composition of a cyclic peptide compound having a water content of 3%-20%, represented by formula I is the structural formula of the cyclic peptide compound, and, also disclosed are a preparation method for same and uses thereof.

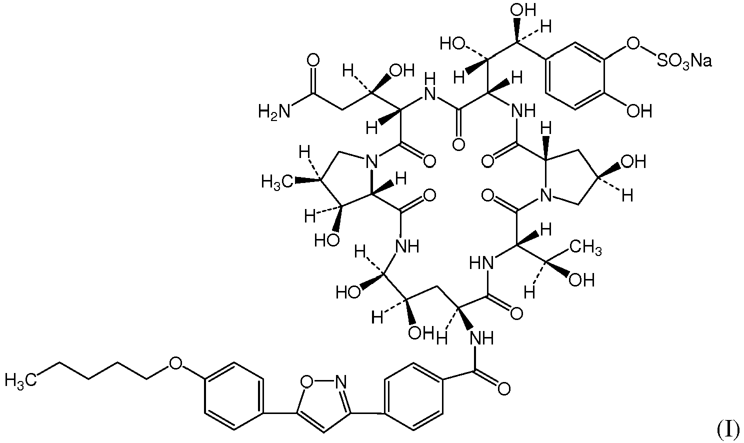

## Description

### Technical field

The present invention relates a composition of a compound, particularly, to a composition of a cyclic peptide compound and water as well as preparation methods and uses thereof.

### Background

Micafungin is a novel anti-fungal drug of pneumocandins, and it inhibits the synthesis of the main ingredient of fungi cell walls, i.e. β-1,3-D-glucan synthase, and therefore destroy the structure of fungal cells, thus leading to cytolysis. Micafungin is widely used for treating various infections, such as infections caused by *Aspergillus, Candida, Cryptococcus, Mucor, Actinomyces, Histoplasma, Dermatophytes* and *Fusarium* and the like.

Micafungin Sodium (also named as FK463) is the active pharmaceutical ingredient of Mycamine. The chemical structure of micafungin Sodium is shown as follows: 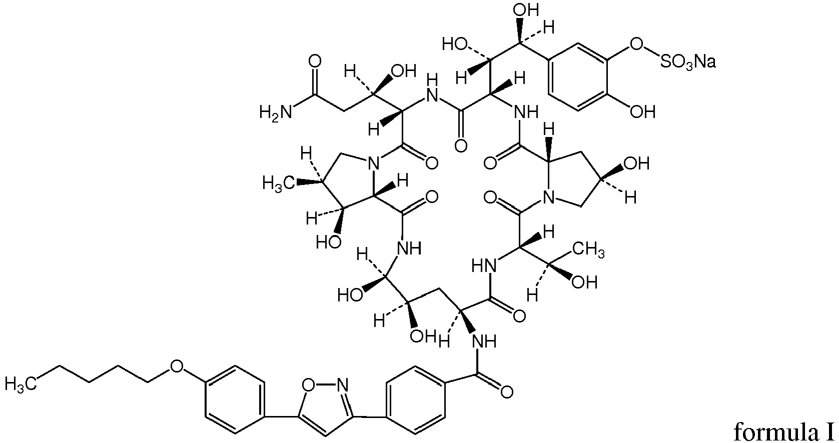 Sodium
5-[(1S,2S)-2-[(3S,6S,9S,11R,15S,18S,20R,21R,24S,25S,26S)-3-[(R)-2-carbamoyl-1-hy droxyethyl]-11,20,21,25-tetrahydroxy-15-[(R)-1-hydroxyethyl]-26-methyl-2,5, 8,14,17,2 3-hexaoxo-18-[4-[5-(4-pentoxyphenyl)isoxazol-3-yl]benzoylamino]-1,4,7,13,16,22-hex aazatricyclo[22.3.0.0^{9,13}]heptacosan-6-yl]-1,2-dihydroxyethyl]-2-hydroxy phenyl sulfate.

The compound of formula I is a polypeptide compound with poor stability, and its quality and efficacy will be affected by degraded products generated during transportation or long-term preservation. And it is difficult to crystallize the compound of formula I, and generally, it is amorphous.

U.S. Patent Nos. 6,107,458 and 7,199,248 and WO 96/111210 disclosed methods for preparing and purifying the compounds of Formula I. Wherein, in U.S. Pat. No. 7,199,248, Micafungin DIPEA (diisopropylethylamine) salt was purified through filtration and chromatographic separation, and then precipitated with acetone and ethyl acetate to give the amorphous form of the compound of formula I.

In Atsushi Ohigashi et al., "Process Development of Micafungin , a Novel Lipopeptide Antifungal Agent", Journal of Synthesit Organic Chemistry, 2006, Vol 64, 12, it was disclosed that the compound of formula I can be precipitated by adding a mixture of acetone and ethyl acetate to the elution solution of the compound of formula I from ion exchange, so as to give the amorphous compound of formula I.

In addition, the patent application WO 03/018615 of Fujisawa Pharmaceutical Co., Ltd. disclosed a new crystal form of the compound of the formula I and a preparation method thereof. In WO03/018615, the compound of formula I in amorphous form was dissolved in an aqueous single alcohol solution or aqueous acetone solution, and a solvent, such as ethyl acetate, methylene chloride, acetone and acetonitrile was added, so as to give needle-like crystals of the compound of formula I of B82 type. The crystal was obtained in an organic solvent, showed needle-like under microscope, and has peaks at the following 2θ angles in the X-ray diffraction pattern: 4.6°, 5.5°, 9.0°, 9.8°, 16.9°.

YAMASHITA et al., from Fujisawa Pharmaceutical Co., Ltd. disclosed ("Study of Industrial Manufacturing Methods for Micafungin (FK463)", Seibutsu kogaku Kaishi, 2005, Vol 83) that needle-like crystals of FK463 were successfully obtained through optimization of solvent and control of pH, however, no specific embodiments and crystal data were disclosed. Since the prior patent application WO03/018615 of the company disclosed B82-type needle-like crystals of the compounds of formula I, it is assumed that YAMASHITA et al. also obtained needle-like crystals of B82 type.

The present inventors prepared needle-like crystals of B82 type according to the method of Example 1 in WO03/018615, and the obtained crystal was observed under an optical microscope, which is about 1 µm in size and a fine needle-like crystal. When exposed to the environment, the crystal of B82 type is conducive to absorption of moisture and has poor stability.

At present, it is disclosed that solids of Micafungin sodium are of poor stability, and can only be stored at a low temperature or a large amount of excipients have to be added to ensure its stability, which greatly limits the development of pharmaceutical uses of Micafungin sodium. If a stable solid of Micafungin sodium can be found, it can be prepared into various different formulations, such as freeze-dried powder, tablets, capsules, ointment, etc., to facilitate the use for different patients.

Therefore, there is an urgent need in the art to obtain a composition of the compound compound of formula I with better stability, thereby achieving better commercial production.

### Summary of the invention

One object of the present invention is to provide a composition of the compound of formula I and water.

Another object of the present invention is to provide preparation methods for the composition of the compound of formula I and water.

Another object of the present invention is to provide uses of the composition of the compound of formula I and water.

### Composition of the compound of formula I and water

In the present invention, a composition of the compound of formula I and water is provided.

In a preferred embodiment of the present invention, a composition of the compound of formula I and water is provided in the present invention.

In a preferred embodiment of the present invention, the water content in the composition is 3% to 20% by weight.

In a preferred embodiment of the present invention, the water content in the composition is 4% to 16% by weight.

In another preferred embodiment of the present invention, HPLC purity of the compound of formula I in the composition is not lower than 98%.

In another preferred embodiment of the present invention, the composition has a maximum peak at 120-130°C on differential scanning calorimetry (DSC) pattern.

As well-known in the art, the drug stability is closely related to the moisture content. It is reported in literatures and books (e.g., "Pharmaceutics") relating to drugs that water is the medium for chemical reaction, and after water is absorbed by a drug in solid form, a liquid film will form on its surface, and hydrolysis or oxidative decomposition reaction will occur in the film. Trace amount of water can accelerate the decomposition of unstable drugs. Moisture content of API, such as ampicillin, should be controlled at a relatively low level, generally about 1%. The higher the moisture content, the faster decomposition goes. After studying the stability of the compound of formula I in amorphous form, the inventors have found that when the moisture content is controlled at not higher than 1%, stability of the compound of formula I is better. However, even if the moisture content of the compound of formula I in amorphous form is not higher than 1%, the compound will significantly degrade after storing for a long time, therefore, requirements on the stability of the compound of formula I in the art can not be satisfied.

After extensive researches, the inventors have found that the moisture content of the composition of the compound of formula I and water has an important effect on the stability of the compound. Even more surprisingly, the inventors have found that high moisture content will effectively improve the stability of the compound of formula I, instead of accelerating the decomposition of the compound and deteriorating the stability of the compound. The stability of the compound at that moisture content is remarkably better than the stability of the compound at other moisture contents, and is better than the stability of the crystal of B82 type disclosed in WO03/018615 and solids in amorphous form.

The inventors have detected and studied the composition of the compound of the formula I and water with different contents of water by DSC, and found that when the content of water in the composition of the compound of formula I is 3%-20%, there is a distinct endothermic peak near 120-130°C in DSC pattern, which indicates that crystalline water is contained in the composition, and it can be also found, from the DSC pattern, that non-crystalline water is also contained in the composition. When the content of water in the composition varies between 3%-20%, there is a change in the non-crystalline water, while no significant change can be found in the crystalline water. Therefore, the composition, the content of water of which is 3%-20%, can always possess excellent stability. When the content of water in the composition of the compound of formula I and water is lower than 3%, according to DSC pattern, it is demonstrated that non-crystalline water is lost in the composition around 105°C, and no crystalline water is contained in the composition, and the composition possesses poor stability. When the content of water in the composition of the compound of formula I and water is higher than 20%, the composition of the compound of formula I and water can not exist in solid form. In the stability test, the inventors have found that, after placed at 25°C for 30 days, the composition of the compound of formula I and water, the content of water in which is not higher than 3%, significantly degraded, and the purity thereof is reduced from 99.52% to 92.18%. While under the same conditions, the purity of the composition of the compound of formula I and water, the content of water in which is not lower than 3%, will not substantially change. Therefore, the compound of formula I in the composition will be stable, only if the content of water in the composition of the compound of formula I and water is controlled within the range of 3% to 20%.

### Identification and characteristics of the composition of the compound of formula I and water

After obtaining the composition of the compound of formula I and water, characteristics of the composition were further studied by the inventors through various means and instruments.

"Differential scanning calorimetry" (DSC) is a technology for measuring the relationship of energy difference and temperature between the tested substance and the reference during the heating process. On the DSC pattern, the location, form and number of the peak are relevant to the properties of the substance; therefore, the substance can be qualitatively identified by using DSC. Said method is used in the art to detect many parameters of a substance, such as the phase transition temperature, glass transition temperature and reaction heat. DSC is known in the art. For example, DSC pattern of a crystal can be obtained by using DSC Q20 differential scanning calorimeter under the following conditions: warming rate of 10°C/min, from 25°C to 300°C. During the detection through DSC, generally, the detected material will lose non-crystalline water at lower than 105°C, and lose crystalline water at higher than 120°C, and there will be distinct endothermic peaks for crystalline water during the detection.

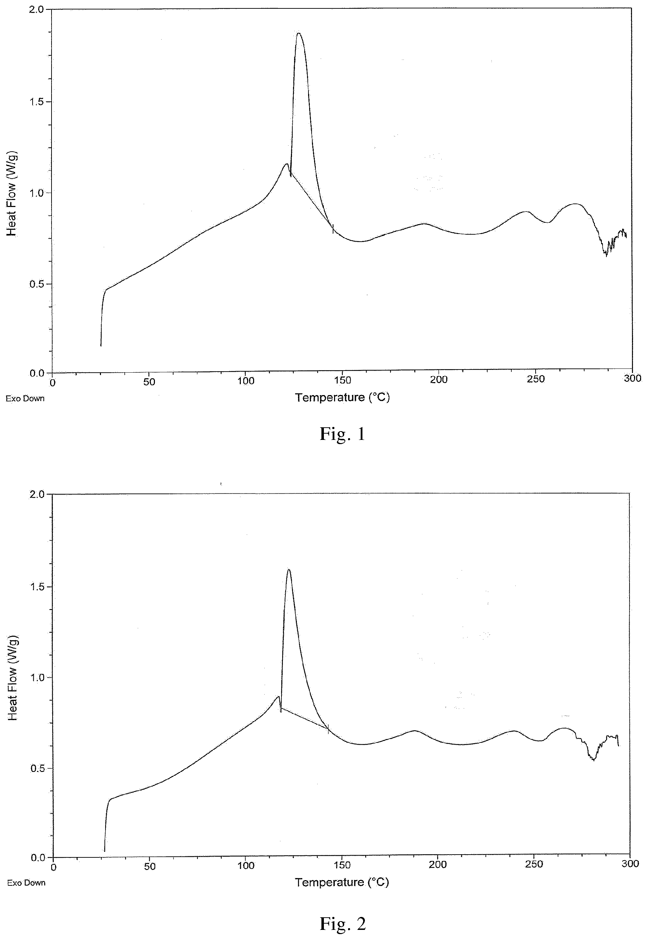

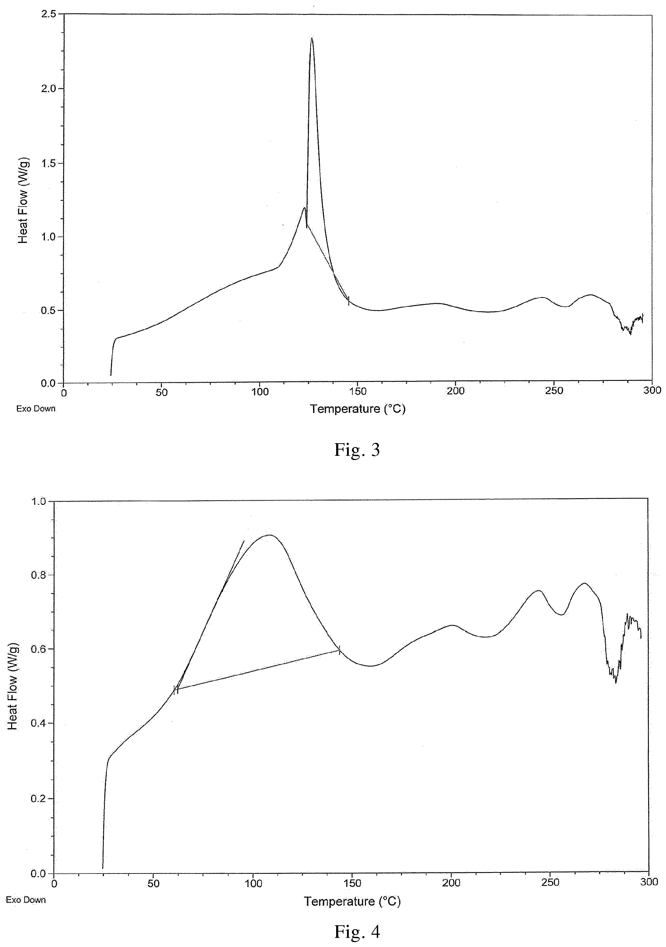

In one embodiment of the present invention, the composition of the compound of formula I and water obtained by the method according to the present invention was determined to have a maximum peak at 120-130°C by DSC. In another embodiment of the present invention, the composition of the compound of formula I and water obtained by the method according to the present invention was determined to have a maximum peak at 129°C by DSC; preferably, the composition has the DSC pattern substantially identical with figure 1. In another embodiment of the present invention, the composition of the compound of formula I and water obtained by the method according to the present invention was determined to have a maximum peak at 123°C by DSC; preferably, the composition has the DSC pattern substantially identical with figure 2. In still another embodiment of the present invention, the composition of the compound of formula I and water obtained by the method according to the present invention was determined to have a maximum peak at around 127°C by DSC; preferably, the composition has the DSC pattern substantially identical with figure 3.

The needle-like crystals of B82 type were prepared by the inventors according to the method of Example 1 of WO03/018615 and detected by DSC, in which there is no significant endothermic peak at 120-130°C.

Amorphous solids of the compound of formula I were prepared by the inventors and detected by DSC, in which there is no significant endothermic peak at 120-130°C.

The content of water in the composition of the compound of formula I is detected by a common method in the art, such as Karl Fischer (KF).

High performance liquid chromatography (HPLC) is a common method for detecting the purity of a compound, wherein a liquid is used as the mobile phase and a high-pressure transfusion system is used for pumping the mobile phase, such as single solvents with different polarities or a mixture of solvents at different proportions, buffers, into a column packed with a stationary phase. Each component is separated in the column, and then enters into a detector for detection, thereby analyzing a sample. In the present invention, HPLC is used for determining the purity of the compound of formula I and studying the stability of a sample. Conditions for HPLC detection are listed as follows:
Analysis Column: YMC-ODS 250 × 4.6 mm, 5 µm;
Mobile phase: acetonitrile : phosphate buffer (pH 3.0) = 45 : 70;
Flow rate: 1 ml/min;
Column temperature: 35°C;
Diluent: aqueous phosphate buffer;
Detection wavelength: 210 nm;
Injection volume: 10 µl.

At present, X-ray powder diffraction, i.e., X-ray polycrystal diffraction (XRD or XRPD), is commonly used as the test method for determining the structure of crystal (i.e., crystal form). X-ray powder diffractometer is used, and a series of diffraction patterns can be produced when X-ray passing through a crystal. In the pattern, different diffraction lines and the intensities thereof are determined by atomic cluster having certain structure, thereby determining the structure of a crystal. The methods for determining the X-ray diffraction pattern of a crystal are known in the art. For example, X-ray diffraction pattern can be obtained by using RIGAKU D/max 2550VB/PC X-ray powder diffractometer with the scanning rate of 2°/min. And copper irradiated target is used.

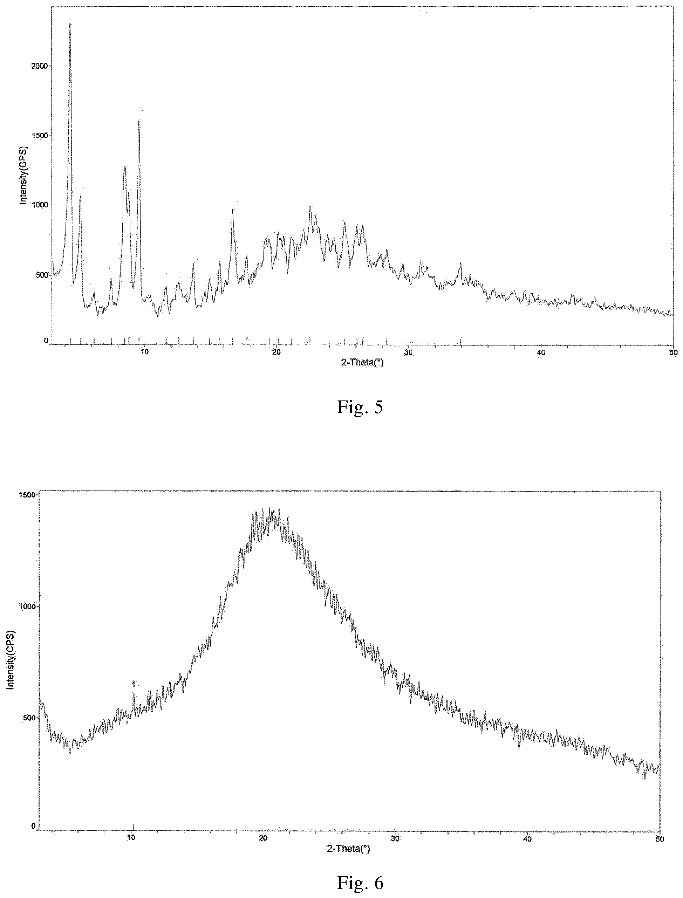

The compound of formula I in the composition of the compound of formula I and water according to the present invention possesses a unique crystal form, and there are specific characteristic peaks in the X-ray diffraction pattern. Particularly, the compound of formula I in the composition of the present invention possesses characteristic peaks at the following 2θ angles in the X-ray powder diffraction pattern: 4.4±0.2°, 5.2±0.2°, 8.5±0.2°, 9.6±0.2°; in a preferred embodiment, there are other characteristic peaks at the following 2θ angles in the pattern: 7.5±0.2°, 8.8±0.2°, 16.6±0.2°, 13.7±0.2°, 22.5±0.2°; in another preferred embodiment, there are other characteristic peaks at the following 2θ angles in the pattern: 12.6±0.2°, 14.9±0.2°, 15.6±0.2°, 25.1±0.2°. In a preferred embodiment, the compound of formula I in the composition of the present invention possesses characteristic peaks at the following 2θ angles in the X-ray powder diffraction pattern: 4.4±0.1°, 5.2±0.1°, 8.5±0.1°, 9.6±0.2°; in another preferred embodiment, there are other characteristic peaks at the following 2θ angles in the pattern: 7.5±0.2°, 8.8±0.1°, 16.6±0.1°, 13.7±0.1°, 22.5±0.1°; in another preferred embodiment, there are other characteristic peaks at the following 2θ angles in the pattern: 12.6±0.1°, 14.9±0.1°, 15.6±0.2°, 25.1±0.1°. More preferably, X-ray diffraction pattern (XRPD) of the compound of formula I in the composition is substantially identical with Fig. 5.

For X-ray powder diffraction method, the state of a substance is identified by comparing the relative intensity of diffraction peaks and the value of mirror spacing d (or 2θ) between drug samples of different crystal forms. Regarding the deviation of 2θ angle for a crystal form, it is stipulated in Japanese Pharmacopoeia that: "for different crystalline forms of the same chemical drug, the allowable deviation of 2θ should be less than ± 0.2°. Relevant provisions also can be found in US Pharmacopoeia (USP27, Page 2401-2402): "The diffraction angle of the sample and the reference should be consistent within the accuracy range of the diffractometer calibration (2θ value should be reproducible, ±0.10°)". Therefore, for two crystals of the same compound, when the deviation of characteristic peak on the X-ray powder diffraction pattern is greater than ± 0.2°, the characteristic peaks will be considered as being different, and the two crystals are of different crystal forms.

The peak at the 2θ reflection angle on the X-ray powder diffraction pattern of the compound of formula I in the composition of the compound of formula I and water according to the present invention is a specific characteristic, which is significantly different from the characteristic peak at the reflection angle on the X-ray powder diffraction pattern of the crystal of the B82 type disclosed in WO03/018615. Absorption intensity and angles of patterns of the compound of formula I in the composition and B82-type crystal are compared as follows: (1) for the compound of formula I in the composition prepared in the present invention, there is a characteristic absorption peak of moderate intensity at 5.1-5.2°, while in X-ray powder diffraction pattern of the crystal of B82 type disclosed in WO03/018615, there is an absorption peak only at 5.5°, and the two characteristic peaks differ by 0.3-0.4°. According to the requirements in Japanese Pharmacopoeia and US Pharmacopoeia and error range of the existing X-ray powder diffractometer which is generally within 0.1°, up to 0.2°, the difference between the two characteristic peaks is not caused by instrument error and the two peaks are indeed different characteristic peaks; (2) for the compound of formula I in the composition prepared in the present invention, there is a strongest characteristic absorption peak at 4.4°, while in X-ray powder diffraction pattern of the crystal of B82 type disclosed in WO03/018615, there is a strongest absorption peak at 9.8°. Therefore, X-ray powder diffraction patterns of the compound of formula I in the composition prepared in the present invention and the crystal of B82 type are different, and the crystals are of different crystal forms.

### Preparation of the composition of the compound of formula I and water

Preparation methods for the composition of the compound of formula I and water are provided in the present invention.

During the study on the compound of formula I, the inventors found that: if only biphasic system is used, the obtained solids are in amorphous form and of poor stability. For obtaining the compound of formula I with good stability, the inventors have screened solvent systems for crystallization by using different solvent combinations in three-phase system. Upon a long period of research, the present inventors have unexpectedly found that can be obtained in a aqueous methanol/isobutanol, methanol/isopropanol, methanol/n-propanol solution, i.e., three-phase system solution or four-phase solvent system by technical means for reducing the solubility of the compound of formula I in a solution, such as reducing the temperature or adding insoluble solvents. The obtained solvate is dried together with a water system to remove organic solvents, so as to obtain a composition of the compound of formula I and water with good stability. The inventors have finally determined the process for preparing the composition of the compound of formula I and water after a large number of solvent screening tests.

A preparation method for the composition of the compound of fomula I and water includes the steps of:
(a) dissolving the compound of formula I in an aqueous mixed solution of alcohols;
(b) obtaining solids by reducing the temperature and / or adding an organic solvent (i);
(c) vacuum-drying the solids obtained in step (b) together with a water system, controlling the content of water, thereby obtaining the composition.

Wherein the mixed solution of alcohols in step (a) is selected from a group consisting of methanol/isobutanol, methanol/isopropanol, methanol/n-propanol.

Wherein, in the aqueous mixed solution of alcohols in step (a), the volume ratio of the two alcohols is 0.01-100, preferably 0.05-20, more preferably 0.1-10.

Wherein, in the aqueous mixed solution of alcohols in step (a), the ratio of total volume of the alcohol to the volume of water is 0.1 to 100, preferably 0.5 to 10, more preferably 1 to 7.

Wherein, the temperature for dissolution in step (a) is 10 - 50°C, preferably, 20-40°C.

Wherein, in step (b), the organic solvent (i) is selected from a group consisting of n-propanol, isopropanol, isobutanol, methyl acetate, ethyl acetate, n-propyl acetate, isopropyl acetate.

Wherein, in step (b), the temperature is reduced to -40 to 35°C, preferably -20 to 35°C, more preferably -10 to 30°C, most preferably -5 to 15°C.

Wherein the volume ratio of organic solvent (i) in step (b) to the aqueous mixed solution of alcohols in step (a) is 0.1 to 50, preferably 0.1 to 10, and more preferably 1-5.

Wherein the water system in step (c) includes tap water, pure water, ice-water mixture or other substance capable of releasing water vapor.
wherein, "vacuum-drying the obtained solids together with a water system" in step (c) means that the solids will be placed in a position where a sample is generally put in a vacuum-dryer, and an open container comprising the substance capable of releasing water vapor is placed around the obtained solids.

Wherein, in step (c), the content of water is controlled at 3%-20%, preferably 4%-16%.

### Uses of the composition of the compound of formula I and water and composition thereof

The composition of the compound of formula I and water provided by the present invention is in a form of API, and can be used in the preparation of a pharmaceutical composition, especially a medicament for treating fungal infections.

### Relevant terms

As used herein, the term "the composition of the compound I and water" and "the composition of the compound of formula I and water" can be interchangeably used, both of which mean a mixture of the compound of formula I and water, wherein water exists as crystalline water and non-crystalline water.

As used herein, the term "crystal" means the solid of a molecule or atom complex showing specific arrangement.

As used herein, "the compound of formula I", "compound I" and "the compound according to formula I" can be interchangeably used, all of which mean a compound of the following structural formula:

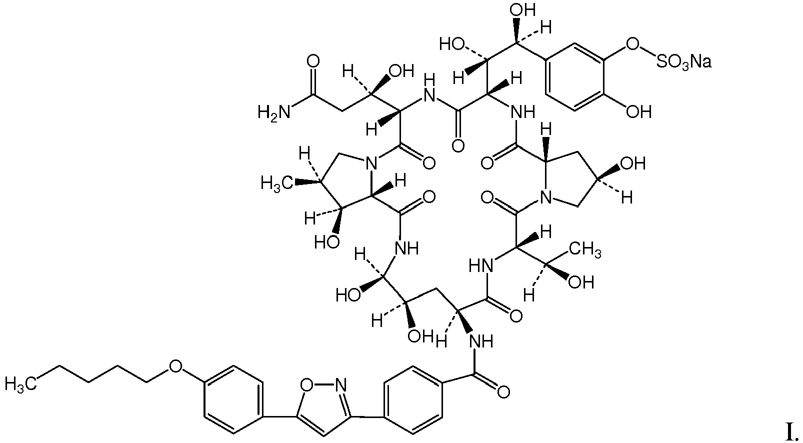

The compound of formula I can be obtained by routine methods in the art, for example (but not limited to), the preparation method disclosed in WO96/11210; alternatively, the compound can be commercially obtained, such as from Fujisawa, Japan.

As used herein, the term "API", as defined in ICH Q7A, refers to any substance or a mixture of substances used in the manufacture of medicaments. And when used in the manufacture of medicaments, it is an active ingredient of the medicament. Such substances have pharmacological activity or other direct effects in diagnosis, treatment, symptom-releasing, treatment or prophylaxis of diseases, or can affect the function or structure of organisms. API refers to raw materials used in the production of various types of formulations, which is the active ingredient in a preparation, but can not be directly taken by a patient. The dry content of major ingredient in API is greater than 90%, preferably greater than 95%, more preferably greater than 98%. The dry content of API refers to the mass percentage of the active ingredient in API after volatile impurities, such as water and residual solvents are removed from API.

As used herein, the term "pharmaceutically acceptable carrier" means the carriers that can be used to administrate therapeutics, including various excipients and diluents. The term means the drug carriers which themselves are not necessary active ingredients, and will not produce undue toxicity upon administration. Suitable carriers are generally known to the skilled in the art. Detailed review regarding the pharmaceutical acceptable excipient can be found in Remington's Pharmaceutical Sciences (Mack Pub. Co., N.J. 1991). Pharmaceutically acceptable excipients in a composition may include liquid, such as water, saline, glycol and ethanol. Additionally, auxiliary substances, such as disintegrating agents, wetting agents, emulsifying agents, pH buffering substances, etc., can be present in the carriers.

The advantages of the invention mainly include:
1. Compositions of the compound of formula I and water with superior stability were provided, which are convenient for transportation and storage, thereby resolving technical problems to be resolved in the prior art.
2. Preparation methods for the compositions of the compound of formula I and water were provided, and such methods are suitable for large-scale production.

### Brief description of the Drawings

Figure 1 shows a DSC pattern of the composition of the compound of formula I and water.
Figure 2 shows a DSC pattern of the composition of the compound of formula I and water.
Figure 3 shows a DSC pattern of the composition of the compound of formula I and water.
Figure 4 shows a DSC pattern of the composition of the compound of formula I and water.
Figure 5 shows the X-ray powder diffraction (XRPD) pattern of the compound of formula I in the composition of the compound of formula I and water; wherein

| No of Peaks | 2-θ | d(A) | I% (Relative intensity) |
|---|---|---|---|
| 1 | 4.4 | 19.8888 | 100.0 |
| 2 | 5.2 | 17.0426 | 46.0 |
| 3 | 7.5 | 11.8100 | 20.3 |
| 4 | 8.5 | 10.3938 | 55.2 |
| 5 | 8.8 | 10.0411 | 46.5 |
| 6 | 9.6 | 9.2244 | 69.7 |
| 7 | 12.6 | 7.0200 | 19.3 |
| 8 | 13.7 | 6.4581 | 25.4 |
| 9 | 14.9 | 5.9329 | 20.4 |
| 10 | 15.7 | 5.6400 | 25.4 |
| 11 | 16.7 | 5.3169 | 41.9 |
| 12 | 22.5 | 3.9443 | 43.0 |
| 13 | 25.1 | 3.5395 | 38.0 |

Figure 6 shows the X-ray powder diffraction (XRPD) pattern of the compound of formula I in amorphous form.

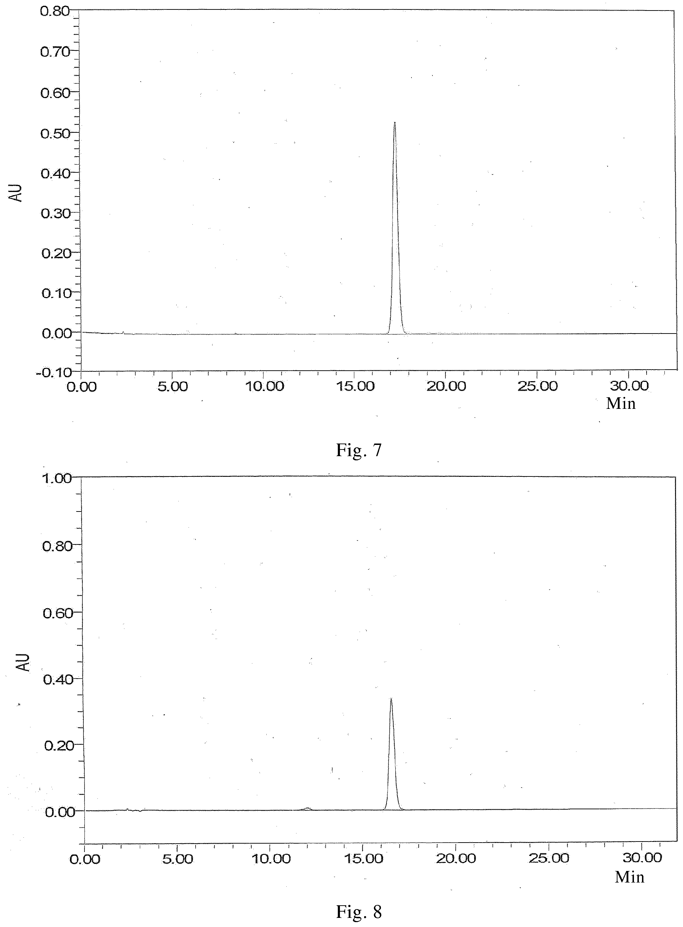

Figure 7 is a HPLC pattern for the composition of the compound of formula I and water obtained in Example 2 after placed at 25°C for 30 days.
Figure 8 is a HPLC pattern for crystal of B82 type obtained in Comparative Example 1 after placed at 25°C for 30 days.

### Mode for carrying out the invention

The invention will be further illustrated with reference to the following specific examples. It is to be understood that these examples are only intended to illustrate the invention, but not to limit the scope of the invention. For the experimental methods in the following examples without particular conditions, they are performed under routine conditions or as instructed by the manufacturer. Unless otherwise specified, all percentages, ratios, proportions or parts are by weight.

The unit of the weight/volume percentages in the invention is well known to the skilled in the art, for example, the weight of a solute in a 100 mL solution.

Unless otherwise defined, all scientific and technical terms used herein have the same meaning as commonly understood by the skilled in the art. Furthermore, any process or material similar or equivalent to those described herein can be used in the process of the present invention. The preferred embodiments and materials described herein are merely provided for illustration.

### Comparative Example 1

### Preparation of the crystal of B82 type

Needle-like crystals of B82 type were obtained according to the method of Example 1 of WO03/018615. The crystals were detected by DSC, in which there is no significant endothermic peak at 120-130°C.

### Example 1

### Preparation of compound I

The amorphous powder of the compound of formula I was prepared according to the method of U.S. Patent No. 7,199,248, and the X-ray powder diffraction pattern thereof is shown in Fig. 6.

### Example 2

### Preparation of the composition of the compound of formula I and water

At 25°C, 1 g of compound of formula I in amorphous form prepared in Example 1 was dissolved into 50 ml of aqueous methanol/isobutanol solution (isobutanol: water: methanol = 8: 2: 1), the obtained solution was cooled to 8°C, solids precipitated from the solution, and the system was stirred for 3.5 hours at this temperature, so that large amount of solids precipitated. 90 ml of ethyl acetate was slowly added, and the solids were obtained by filtration. The obtained solids were placed into a vaccum-drying oven, a plate of tap water was put on the bottom of the vacuum-dryer, and the content of water was controlled at 9.1%. The composition of the compound of formula I and water was obtained by vaccum-drying, and the purity of the compound of formula I was detected by HPLC at 99.53%. DSC and XRPD patterns can be found in Figures 1 and 5.

### Example 3

### Preparation of the composition of the compound of formula I and water

At 30°C, 2.5 g of crystals of B82 type prepared in Comparative Example 1 was dissolved into 50 ml of aqueous methanol/isobutanol solution (isobutanol: water: methanol = 1: 1: 1), 50 ml of methyl acetate was slowly added, and solids were obtained by filtration. The obtained solids were placed into a vaccum-drying oven, a plate of pure water was put on the bottom of the vacuum-dryer, and the content of water was controlled at 16%. The composition of the compound of formula I and water was obtained by vaccum-drying, and the purity of the compound of formula I was detected by HPLC at 99.5%.

### Example 4

### Preparation of the composition of the compound of formula I and water

At 10°C, 3 g of the compound of formula I in amorphous form prepared in Example 1 was dissolved into 600 ml of aqueous methanol/isobutanol solution (isobutanol: water: methanol = 5: 1: 2), the obtained solution was cooled to -20°C, solids precipitated from the solution, the system was stirred for 2 hours, a large amount of solids precipitated, and the solids were obtained by filtration. The obtained solids were placed into a vaccum-drying oven, a plate of trash ice was put on the bottom of the vacuum-dryer, and the content of water was controlled at 3%. The composition of the compound of formula I and water was obtained by vaccum-drying, and the purity of the compound of formula I was detected by HPLC at 99.61%. DSC pattern can be found in Figure 2.

### Example 5

### Preparation of the composition of the compound of formula I and water

At 50°C, 3 g of the compound of formula I in amorphous form prepared in Example 1 was dissolved into 120 ml of aqueous methanol/isopropanol solution (isopropanol: water: methanol = 1: 4: 1), the obtained solution was cooled to 30°C, solids precipitated from the solution, the system was stirred for 30 mins, a large amount of solids precipitated, 200 ml of isopropanol was slowly added, and the solids were obtained by filtration. The obtained solids were placed into a vaccum-drying oven, a plate of pure water was put on the bottom of the vacuum-dryer, and the content of water was controlled at 20%. The composition of the compound of formula I and water was obtained by vaccum-drying, and the purity of the compound of formula I was detected by HPLC at 99.64%. DSC pattern can be found in Figure 3.

### Example 6

### Preparation of the composition of the compound of formula I and water

At 20°C, 1 g of the compound of formula I in amorphous form prepared in Example 1 was dissolved into 20 ml of aqueous methanol/isopropanol solution (isopropanol: water: methanol = 10: 2: 1), 200 ml of methyl acetate was slowly added, and the solids were obtained by filtration. The obtained solids were placed into a vaccum-drying oven, a plate of tap water was put on the bottom of the vacuum-dryer, and the content of water was controlled at 18.3%. The composition of the compound of formula I and water was obtained by vaccum-drying, and the purity of the compound of formula I was detected by HPLC at 99.63%.

### Example 7

### Preparation of the composition of the compound of formula I and water

At 18°C, 1.0 g of the compound of formula I in amorphous form prepared in Example 1 was dissolved into 100 ml of aqueous methanol/isopropanol solution (isopropanol: water: methanol = 1: 2: 20), the obtained solution was cooled to -5°C, solids precipitated from the solution, the system was stirred for 4 hours, a large amount of solids precipitated, and the solids were obtained by filtration. The obtained solids were placed into a vaccum-drying oven, a plate of ice-water mixture was put on the bottom of the vacuum-dryer, and the content of water was controlled at 12.3%. The composition of the compound of formula I and water was obtained by vaccum-drying, and the purity of the compound of formula I was detected by HPLC at 99.65%.

### Example 8

### Preparation of the composition of the compound of formula I and water

At 30°C, 2 g of the compound of formula I in amorphous form prepared in Example 1 was dissolved into 20 ml of aqueous methanol/n-propanol solution (n-propanol: water: methanol = 1: 15: 10), the obtained solution was cooled to -15°C, solids precipitated from the solution, the system was stirred for 2 hours, a large amount of solids precipitated, 100 ml of isopropyl acetate was slowly added, and the solids were obtained by filtration. The obtained solids were placed into a vaccum-drying oven, a plate of pure water was put on the bottom of the vacuum-dryer, and the content of water was controlled at 6.3%. The composition of the compound of formula I and water was obtained by vaccum-drying, and the purity of the compound of formula I was detected by HPLC at 99.64%.

### Example 9

### Preparation of the composition of the compound of formula I and water

At 25°C, 4 g of the compound of formula I in amorphous form prepared in Example 1 was dissolved into 300 ml of aqueous methanol/n-propanol solution (n-propanol: water: methanol = 20: 2: 1), 30 ml of isobutanol was slowly added, and the solids were obtained by filtration. The obtained solids were placed into a vaccum-drying oven, a plate of pure water was put on the bottom of the vacuum-dryer, and the content of water was controlled at 3.7%. The composition of the compound of formula I and water was obtained by vaccum-drying, and the purity of the compound of formula I was detected by HPLC at 99.42%.

### Example 10

### Preparation of the composition of the compound of formula I and water

At 40°C, 2.7 g of the compound of formula I in amorphous form prepared in Example 1 was dissolved into 80 ml of aqueous methanol/n-propanol solution (n-propanol: water: methanol = 10: 3: 1), the obtained solution was cooled to -10°C, solids precipitated from the solution, the system was stirred for 1 hour, a large amount of solids precipitated, and the solids were obtained by filtration. The obtained solids were placed into a vaccum-drying oven, a plate of ice-water mixture was put on the bottom of the vacuum-dryer, and the content of water was controlled at 4%. The composition of the compound of formula I and water was obtained by vaccum-drying, and the purity of the compound of formula I was detected by HPLC at 99.58%.

### Example 11

### Preparation of the composition of the compound of formula I and water

At 20°C, 1.5 g of the compound of formula I in amorphous form prepared in Example 1 was dissolved into 70 ml of aqueous methanol/isobutanol solution (isobutanol: water: methanol = 8: 2: 1), the obtained solution was cooled to 0°C, crystals precipitated from the solution, the system was stirred for 4.5 hours at this temperature, a large amount of solids precipitated, 100 ml of ethyl acetate was slowly added, and the solids were obtained by filtration. The obtained solids were placed into a vaccum-drying oven, a plate of pure water was put on the bottom of the vacuum-dryer, and the content of water was controlled at 8.9%. The composition of the compound of formula I and water was obtained by vaccum-drying, and the purity of the compound of formula I was detected by HPLC at 99.63%.

### Comparative Example 2

### Preparation of compositions of the compound of formula I and water with different contents of water

At 50°C, 3 g of the compound of formula I in amorphous form prepared in Example 1 was dissolved into 120 ml of aqueous methanol/isopropanol solution (isopropanol: water: methanol = 4: 2: 1), the obtained solution was cooled to 30°C, solids precipitated from the solution, the system was stirred for 30 mins, a large amount of solids precipitated, 200 ml of isopropanol was slowly added, and the solids were obtained by filtration. The obtained solids were placed into a vaccum-drying oven, a plate of pure water was put on the bottom of the vacuum-dryer, and the content of water was controlled at 23.5%. The composition of the compound of formula I and water was obtained by vaccum-drying, and the composition was in a semi-liquid state.

### Comparative Example 3

### Preparation of compositions of the compound of formula I and water with different contents of water

At 30°C, 2 g of the compound of formula I in amorphous form prepared in Example 1 was dissolved into 20 ml of aqueous methanol/n-propanol solution (n-propanol: water: methanol = 1: 3: 2), the obtained solution was cooled to 15°C, crystals precipitated from the solution, the system was stirred for 2 hours, a large amount of solids precipitated, 100 ml of isopropyl acetate was slowly added, and the solids were obtained by filtration. The obtained solids were placed into a vaccum-drying oven, a plate of pure water was put on the bottom of the vacuum-dryer, and the content of water was controlled at 2.3%. The composition of the compound of formula I and water was obtained by vaccum-drying. DSC pattern can be found in Figure 4.

### Comparative Example 4

### Preparation of compositions of the compound of formula I and water with different contents of water

At 45°C, 2.7 g of the compound of formula I in amorphous form prepared in Example 1 was dissolved into 80 ml of aqueous methanol/n-propanol solution (n-propanol: water: methanol = 8: 3: 1), the obtained solution was cooled to 10°C, solids precipitated from the solution, the system was stirred for 1 hour, a large amount of solids precipitated, and the solids were obtained by filtration. The obtained solids were placed into a vaccum-drying oven, a plate of ice-water mixture was put on the bottom of the vacuum-dryer, and the content of water was controlled at 27.3%. The composition of the compound of formula I and water was obtained by vaccum-drying, and the composition was in a semi-liquid state.

### Comparative Example 5

### Preparation of compositions of the compound of formula I and water with different contents of water

At 25°C, 1 g of the compound of formula I in amorphous form prepared in Example 1 was dissolved into 50 ml of aqueous methanol/isobutanol solution (isobutanol: water: methanol = 8: 2: 1), the obtained solution was cooled to 8°C, solids precipitated from the solution, the system was stirred for 3.5 hours at this temperature, a large amount of solids precipitated, 90 ml of ethyl acetate was slowly added, and the solids were obtained by filtration. The obtained solids were placed into a vaccum-drying oven, a plate of tap water was put on the bottom of the vacuum-dryer, and the content of water was controlled at 1.1%. The composition of the compound of formula I and water was obtained by vaccum-drying.

### Comparative Example 6

According to the method of Example 2, at 25°C, 1 g of the compound of formula I in amorphous form prepared in Example 1 was dissolved into 50 ml of methanol/water solution (methanol: water = 3: 2), the obtained solution was cooled to 8°C, solids precipitated from the solution, the system was stirred for 3.5 hours at this temperature, a large amount of solids precipitated, 90 ml of ethyl acetate was slowly added, and the solids were obtained by filtration. The obtained solids were placed into a vaccum-drying oven, a plate of tap water was put on the bottom of the vacuum-dryer, and the content of water was determined as 0.8%. The obtained solids were in amorphous form as determined by XRPD, and there is no significant endothermic peak at 120-130°C as determined by DSC.

Solids were prepared according to the above methods by using different solvents and detected by XRPD, and the results are shown in the following table:

| No. | Solvent | Structure of the obtained solid |
|---|---|---|
| 1 | Methanol: water = 3: 2 | Amorphous |
| 2 | Ethanol: water = 5: 1 | Amorphous |
| 3 | Isopropanol: water = 2: 3 | Amorphous |
| 4 | Isobutanol: water = 4: 1 | Amorphous |
| 5 | n-butanol: water = 9: 1 | Amorphous |
| 6 | Acetone: water = 4: 1 | Amorphous |
| 7 | Acetonitrile: water = 3: 1 | Amorphous |
| 8 | Methanol: Ethanol: water = 8: 2: 1 | Amorphous |
| 9 | Propanol: butanol: water = 6: 5: 3 | Amorphous |
| 10 | Methanol: butanol: water = 1: 7: 2 | Amorphous |
| 11 | Ethanol: butanol: water = 2: 2: 5 | Amorphous |
| 12 | Methanol: acetonitrile: water = 4: 1: 2 | Amorphous |
| 13 | Methanol: Ethanol: water = 9: 2: 2 | Amorphous |

### Example 12

### Purity and stability test

In this Example, the purity and stability of samples obtained in Comparative Examples and Examples were compared. The used method is described as follows:
Samples of Examples 1-11 and Comparative examples 1-6 were taken and sealed at 25°C for 30 days respectively. And then the content of impurities in the sample was analyzed.

Results for comparing the stability of the composition of the compound of formula I and water according to the present invention, the crystal of B82 type and the amorphous solids are shown in the following table:

| Sample | Form | Purity of initial sample | Purity of sample after stored ar 25°C for 30 days |
|---|---|---|---|
| Example 2 | Composition of the compound of formula I and water | 99.53% | 99.5% |
| Comparative Example 1 | Crystal of B82 type | 99.50% | 96.98% |
| Comparative Example 6 | Amorphous | 99.38% | 89.27% |

Results for comparing the stability of the composition of the compound of formula I and water with different contents of water according to the present invention are shown in the following table:

| Sample | Content of water | Purity of initial sample | Purity of sample after stored ar 25°C for 30 days |
|---|---|---|---|
| Example 2 | 9.1% | 99.53% | 99.50% |
| Example 3 | 16% | 99.5% | 99.37% |
| Example 4 | 3% | 99.61% | 99.03% |
| Example 5 | 20% | 99.64% | 99.31% |
| Example 6 | 18.3% | 99.63% | 99.37% |
| Example 7 | 12.3% | 99.65% | 99.60% |
| Example 8 | 6.3% | 99.64% | 99.59% |
| Example 9 | 3.7% | 99.42% | 99.2% |
| Example 10 | 4% | 99.58% | 99.45% |
| Example 11 | 8.9% | 99.63% | 99.61% |
| Comparative Example 2 | 23.5% | 99.66% | 95.42% |
| Comparative Example 3 | 2.3% | 99.61% | 94.33% |
| Comparative Example 4 | 27.3% | 99.53% | 93.48% |
| Comparative Example 5 | 1.1% | 99.52% | 92.18% |

From the above data, it is clear that the stability of the composition of the compound of formula I and water with the content of water at 3%-20% is superior to that of the crystal of B82 type, and is superior to that of the amorphous solid. The content of water in the composition of the compound of formula I and water will have significant effects on the stability of the composition, wherein after storing for a long time, compared with the composition of the compound of formula I and water with the content of water hight than 20% or lower than 3%, the composition with the content of water of 3%-20% will have excellent stability.

### Example 12

### Preparation of pharmaceutical composition

| Composition of the compound of formula I and water | Lactose | Anhydrous citric acid | Sodium hydroxide |
|---|---|---|---|
| 2.5g | 20g | q.s. | q.s. |

20 g of lactose was dissolved in purified water (200 ml) by heating at less than 50°C. After cooling to 20°C or lower, 2.5 g of the composition of the compound of formula I and water obtained according to the method in Example 2 was added to the lactose solution, and gently agitated to avoid generation of bubbles. 2% aqueous citric acid solution (0.95 ml) was added, 0.4% aqueous sodium hydroxide solution (about 24 ml) was added to the solution to adjust pH 5.5, and then diluted with pure water to give a volume of 250 ml. The resulting solution was dispensed into 100 vials of 10 ml volume, 2.5 ml per vial. The solution in each vial was lyophilized through a conventional method using a lyophilizer to obtain a pharmaceutical composition, each containing 25 mg of the composition of the compound of formula I and water.

### Example 13

### Preparation of pharmaceutical composition

0.2 g of the compound of the compound of formula I and water obtained by the method in Example 2 was taken and prepared into eye drop according to the method in Example 2 of US2007249546A1.

The above mentioned embodiments are preferred embodiments of the present invention, and not provided to limit the scope of substantial technical contents of the present invention, which are broadly defined in the claims of the present application. If any technical entity or method completed by other people is identical with that defined by the claims of the present application, or is an equivalent modification, all of them will be deemed as falling within the scope of the claims.

## Claims

1. A composition of the compound of formula I and water, wherein the water content in the composition is 3% to 20% by weight;

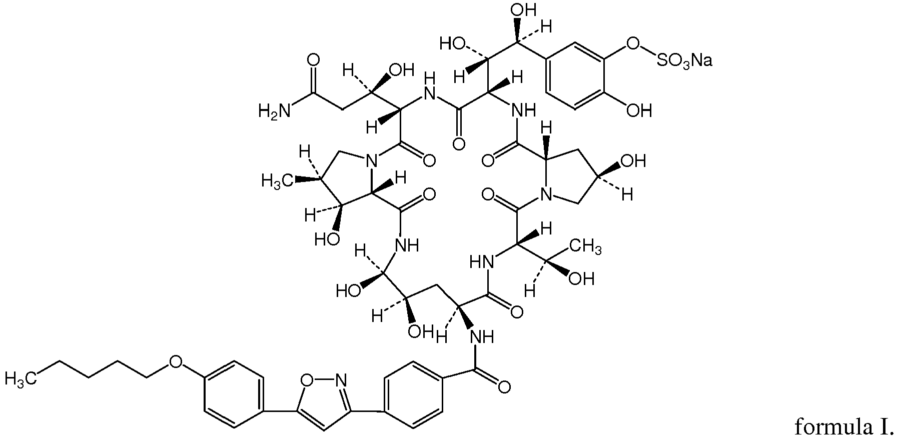

2. The composition of claim 1, wherein the water content in the composition is 4% to 16% by weight.

3. The composition of claim 1, wherein HPLC purity of the compound of formula I in the composition is not lower than 98%.

4. A preparation method for the composition of any one of claims 1-3, including the steps of:
(a) dissolving the compound of formula I in an aqueous mixed solution of alcohols;
(b) obtaining solids by reducing the temperature and / or adding an organic solvent (i);
(c) vacuum-drying the solids obtained in step (b) together with a water system, controlling the content of water, thereby obtaining the composition of any one of claims 1-3.

5. The preparation method of claim 4, wherein the mixed solution of alcohols in step (a) is selected from a group consisting of methanol/isobutanol, methanol/isopropanol, methanol/n-propanol.

6. The preparation method of claim 5, wherein, in the aqueous mixed solution of alcohols in step (a), the volume ratio of the two alcohols is 0.01-100, preferably 0.05-20, more preferably 0.1-10.

7. The preparation method of claim 4, wherein, in the aqueous mixed solution of alcohols in step (a), the ratio of total volume of the alcohol to the volume of water is 0.1 to 100, preferably 0.5 to 10, more preferably 1 to 7.

8. The preparation method of claim 4, wherein, in step (b), the organic solvent (i) is selected from a group consisting of n-propanol, isopropanol, isobutanol, methyl acetate, ethyl acetate, n-propyl acetate, isopropyl acetate.

9. The preparation method of claim 4, wherein, in step (b), the temperature is reduced to -40 to 35°C, preferably -20 to 35°C, more preferably -10 to 30°C, most preferably -5 to 15°C.

10. The preparation method of claim 4, wherein, the volume ratio of organic solvent (i) in step (b) to the aqueous mixed solution of alcohols in step (a) is 0.1 to 50, preferably 0.1 to 10, and more preferably 1-5.

11. The preparation method of claim 4, wherein, the water system in step (c) includes tap water, pure water, ice-water mixture or other substance capable of releasing water vapor.

12. The preparation method of claim 4, wherein, in step (c), the content of water is controlled at 3%-20%.

13. The preparation method of claim 12, wherein, in step (c), the content of water is controlled at, preferably 4%-16%.

14. Use of the composition of any one of claims 1-3 in the preparation of medicaments for treating fungal infections.

15. A pharmaceutical composition comprising the composition of any one of claims 1-3 and a pharmaceutically acceptable carrier.

16. A preparation method for the pharmaceutical composition of claim 15, including the step of:
mixing the composition of any one of claims 1-3 and a pharmaceutically acceptable carrier, thereby obtaining the pharmaceutical composition of claim 15.

17. The composition of claim 1, wherein the composition has a maximum peak at 120-130°C on differential scanning calorimetry (DSC) pattern.

18. The composition of claim 1, wherein the compound of formula I in the composition exists as a crystalline form.

19. The composition of claim 18, wherein the compound of formula I existing as a crystalline form possesses characteristic peaks at the following angles in the X-ray powder diffraction pattern: 4.4±0.2°, 5.2±0.2°, 8.5±0.2°, 9.6±0.2°.

20. The composition of claim 19, wherein the compound of formula I existing as a crystalline form further possesses characteristic peaks at the following angles in the X-ray powder diffraction pattern: 7.5±0.2°, 8.8±0.2°, 16.6±0.2°, 13.7±0.2°, 22.5±0.2°.

21. The composition of claim 20, wherein the compound of formula I existing as a crystalline form further possesses characteristic peaks at the following angles in the X-ray powder diffraction pattern: 12.6±0.2°, 14.9±0.2°, 15.6±0.2°, 25.1±0.2°.

22. The composition of claim 19, wherein the compound of formula I existing as a crystalline form further possesses characteristic peaks at the following angles in the X-ray powder diffraction pattern: 4.4±0.1°, 5.2±0.1°, 8.5±0.1°, 9.6±0.1°.

23. The composition of claim 22, wherein the compound of formula I existing as a crystalline form further possesses characteristic peaks at the following angles in the X-ray powder diffraction pattern: 7.5±0.1°, 8.8±0.1°, 16.6±0.1°, 13.7±0.1°, 22.5±0.1°.

24. The composition of claim 23, wherein the compound of formula I existing as a crystalline form further possesses characteristic peaks at the following angles in the X-ray powder diffraction pattern: 12.6±0.1°, 14.9±0.1°, 15.6±0.1°, 25.1±0.1°.

25. The composition of claim 1, wherein the dry content of the compound of formula I in the composition is not less than 98%.

26. The composition of claim 2, wherein the composition is API.
